# EUROPEAN PATENT SPECIFICATION

(11) **EP 3 221 009 B1**
(45) Date of publication and mention of the grant of the patent: **06.11.2019**
(21) Application number: 15823456.7
(22) Date of filing: 17.12.2015
(51) Int. Cl.: C25B 1/28, C25B 9/06, A61Q 11/00, A46B 15/00, A61N 1/44, A61K 8/23, A61N 1/05, A61C 19/06

(54) **NOVEL PRODUCTS AND METHODS**
NEUARTIGE PRODUKTE UND VERFAHREN
NOUVEAUX PRODUITS ET PROCÉDÉS

(30) Priority: 24.12.2014 US 201462096578 P
(43) Date of publication of application: 27.09.2017
(73) Proprietor: Colgate-Palmolive Company, New York, NY 10022 (US)
(72) Inventor: JOHANSSON, Patrik, Hoboken, New Jersey 07030 (US); XU, Guofeng, Plainsboro, New Jersey 08536 (US)
(74) Representative: Wibbelmann, Jobst
(86) International application number: PCT/US2015/066331
(87) International publication number: WO 2016/106075

(56) References cited:
- WO-A1-2008/001388
- IL-A- 176 647
- US-A- 3 337 466
- US-A1- 2005 069 503
- US-A1- 2006 013 778
- US-A1- 2006 169 949
- US-B1- 6 274 122

## Description

### FIELD

Provided is an oral care product comprising an effective amount of a soluble and orally acceptable sulfate, e.g., sodium, potassium or ammonium bisulfate, in a buffered, electrically conductive medium, which product is useful in a method for *in situ* production of persulfate (S₂O₈²⁻), and methods of making and using the same, for example for whitening the teeth, for oxidizing volatile sulfur compounds that give rise to halitosis, or for killing bacteria on the teeth.

### BACKGROUND

Various products and procedures have been developed to whiten teeth. These products and procedures are either purchased and/or used directly by the consumer or are applied by a dentist or other professional.

Potassium persulfate (K₂S₂O₈) has been proposed to whiten teeth, but due to the high reactivity of potassium persulfate in aqueous environments, it has poor compatibility with common ingredients used in oral care products, and, is therefore difficult to formulate into a stable product. Anhydrous and/or hydrophobic formulations may improve the stability of potassium persulfate, but may interfere with quick release of the persulfate from the formulation matrix, resulting in poor bleaching performance.

Electrochemical methods for treating teeth have been proposed in European Patent No. 1525857 and U.S. Patent No. 7,775,795. Those references, however, do not discuss using persulfate to whiten teeth or the challenges associated with using it as such.

The present disclosure is directed to overcoming one or more problems set forth above and/or other problems of the prior art.

### BRIEF SUMMARY

Provided is an oral care product comprising an effective amount of a soluble and orally acceptable sulfate, e.g., a bisulfate, e.g., sodium, potassium or ammonium bisulfate, in a buffered, electrically conductive medium, which product is useful in a method for *in situ* production of persulfate (S₂O₈²⁻), and methods of making and using the same, for example for whitening the teeth, for oxidizing volatile sulfur compounds that give rise to halitosis, or for killing bacteria on the teeth. The product comprises a cathode and an anode which are electrically connectable to a voltage source, e.g., via a switch.

Also provided is a method for whitening the teeth, for oxidizing volatile sulfur compounds that give rise to halitosis, or for killing bacteria on the teeth, by applying an effective amount of an oral care composition comprising a an orally acceptable sulfate and/or bisulfate, e.g., potassium bisulfate (KHSO₄), in a buffered, electrically conductive medium to the teeth, and (just before or during application) exposing the composition to an electric potential so as to facilitate *in situ* production of persulfate (S₂O₈²⁻), e.g., potassium persulfate (K₂S₂O₈).

Further areas of applicability of the present invention will become apparent from the detailed description provided hereinafter.

### DETAILED DESCRIPTION

The following description of the preferred embodiments is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The term "sulfate" as used herein, means a salt or mixture of salts formed or capable of being formed by reaction of sulfuric acid with a base. The term therefore includes bisulfate salts. In aqueous solutions, there may be an equilibrium between the fully deprotonated ion (sulfate or SO₄²⁻) and the partially deprotonated ion (bisulfate or HSO₄⁻), and both of these ions are capable of forming persulfate when exposed to an electrical potential in an aqueous medium. "Orally acceptable sulfate" refers to sulfates (including bisulfates) which are not toxic or harmful when administered as a component of an oral care product, at relevant concentrations, e.g., 10% or less. "Soluble sulfate" refers to sulfate salts (including bisulfates) which are soluble in aqueous solution at room temperature, e.g., having a solubility of at least 10 g per 100 mL water at 25°C. The term "soluble and orally acceptable sulfate" thus encompasses, for example, compounds such as NaHSO₄, KHSO₄, (NH₄)HSO₄, Mg(HSO₄)₂, Na₂SO₄, K₂SO₄, (NH₄)₂SO₄, and MgSO₄.

As used herein, *"in situ"* means that the persulfate is generated in the oral care product just before or during use and is not added as a separate ingredient.

As used herein, "effective amount" means an amount or concentration effective to perform the desired function when the product is used. Thus an effective amount of sulfate refers to an amount of a sulfate salt (e.g., selected from sodium bisulfate, potassium bisulfate, ammonium bisulfate, magnesium bisulfate, sodium sulfate, potassium sulfate, ammonium sulfate, magnesium sulfate, and mixtures thereof) which, when activated by an electrical current to form persulfate, provides persulfate in an amount or concentration effective to whiten the teeth.

In one embodiment, the activation of the sulfate to form persulfate is carried out just prior to use. For example, oral care product comprising an effective amount of a soluble and orally acceptable sulfate in a buffered, electrically conductive medium is dispensed into a container comprising electrodes, which are linked to a battery or other voltage source to provide an electrical current through the medium to generate persulfate.

If the oral care product is a mouthwash, it may for example be dispensed into the cap of the bottle, where the cap contains the electrodes on the inner surface. A mouthwash product may be poured into the cap, or it may be dispensed via a tube into the cap using a pump or squeezing mechanism, or it may be dispensed in a cap using a dosage system, e.g. by turning the bottle upside down to allow the mouthwash to flow into the cap, then turning the bottle right side up, so that the valve closes and the mouthwash portion to be activated is isolated in the cap chamber. Or the mouthwash may be dispensed into a container, and electricity provided by a toothbrush comprising electrodes or stirrer comprising electrodes, which can be inserted into the formulation to activate the persulfate formation just prior to use.

If the oral care product is a gel, it may for example be dispensed into a dental tray comprising electrodes and shaped to fit over the teeth, so that the persulfate can be generated in the tray just before the tray is applied to the teeth.

In another embodiment, the activation of the sulfate to form persulfate is carried out during use. For example, oral care product comprising an effective amount of a soluble and orally acceptable sulfate in a buffered, electrically conductive medium may be in the form of an aqueous gel which is tray comprising electrodes, which are linked to a battery or other voltage source to provide an electrical current through the medium to generate persulfate in situ, while the tray is in the mouth. Or the gel may be used like a toothpaste, and brushed using a toothbrush comprising electrodes which provide current to generate the persulfate in the mouth during brushing.

For example, in one embodiment, a toothbrush is used to activate the oral care product, the toothbrush comprising an effective amount of a soluble and orally acceptable sulfate in a buffered, electrically conductive medium is a powered toothbrush which includes a handle, a power source, a head including a cavity disposed at a distal end of the handle, a pair of electrodes disposed in the cavity and electrically connected to the power source, and a movable cleaning element connected to the head and movable relative to the cavity. The movable cleaning element includes a bristle support member disposed at least partially over the cavity, a plurality of bristles extending from the bristle support in a direction away from the cavity, and a ferromagnetic member. Application of an electrical current to the electrodes generates a magnetic field at the electrodes, which can be used (i) to move the ferromagnetic element, creating good distribution of the gel and also (ii) to generate the persulfate.

In another embodiment, a toothbrush comprising electrodes is used to activate the soluble and orally acceptable sulfate in a buffered, electrically conductive medium, wherein the toothbrush is as described in Colgate's US 815 6602.

In some embodiments, the polarity of the electrodes is altered at regular intervals during use. In the case of the above toothbrush, alternating the polarity of the electrodes during use changes the magnetic field so each electrode can alternately attract and repel the ferromagnetic member so as to move the movable cleaning element relative to the electrode. But more generally, alternating the polarity of the electrodes prevents accumulation of charged particles or ions on the electrodes, which could interfere with their effectiveness in generating persulfate.

In one embodiment therefore, the disclosure provides an oral care product (Product 1) comprising an effective amount of a soluble and orally acceptable sulfate in a buffered, electrically conductive medium, e.g.,
1.1. Product 1 wherein the sulfate is a salt or mixture of salts selected from sodium, potassium and ammonium salts, e.g., wherein the sulfate is selected from sodium bisulfate, potassium bisulfate, ammonium bisulfate, magnesium bisulfate, sodium sulfate, potassium sulfate, ammonium sulfate, magnesium sulfate, and mixtures thereof.
1.2. Product 1.1 wherein the sulfate is a bisulfate, e.g., selected from sodium bisulfate, potassium bisulfate, ammonium bisulfate, magnesium bisulfate, and combinations thereof, e.g., potassium bisulfate (KHSO₄) and/or sodium bisulfate (NaHSO₄), for example sodium bisulfate (NaHSO₄), e.g., in an amount of 0.1 - 30% e.g., 1-10%, e.g., 2-4%.
1.3. Product 1 or 1.1 wherein the sulfate is capable of being oxidized to persulfate.
1.4. Any foregoing product wherein the electrically conductive medium is an aqueous gel.
1.5. Any foregoing product wherein the electrically conductive medium is an aqueous gel comprising water, e.g., 60-70%, glycerin, e.g. 15-20%, sodium bisulfate, e.g. 5-15%, buffer, e.g., citrate or phosphate buffer, and thickener, e.g., selected from cross-linked polyacrylic acid polymers (e.g., Carbopol), polysaccharides (e.g., xanthan gum), fumed silica, polyvinyl pyrrolidone, carboxymethyl cellulose, gellan gum, and combinations thereof.
1.6. Any foregoing product wherein the electrically conductive medium is an aqueous salt solution, e.g., a phosphate buffer solution.
1.7. Any foregoing product wherein the product further comprises at least two electrodes electrically connectable (optionally via a switch) to a voltage source, wherein the electrodes comprise a cathode and an anode.
1.8. Any foregoing product comprising at least two electrodes electrically connected to a voltage source, such that the electrodes comprise an anode and a cathode and wherein the electrodes comprise one or more conductive materials, e.g., a metal or polymer, e.g., wherein the pairs of electrodes comprise one or more of a conductive element, e.g., selected from carbon, platinum, iron, zinc, copper, aluminum, silver, nickel, and gold, e.g., one or more of platinum, carbon, and iron, e.g., platinum, e.g., carbon, e.g., iron, or wherein the one or more pairs of electrodes comprise a semiconductor material, e.g., titanium dioxide or boron doped diamond.
1.9. Any foregoing product 1.7-1.8 wherein persulfate is synthesized when an electrical potential is applied to the electrodes, e.g., by connection to a voltage source.
1.10. Any foregoing product wherein persulfate is synthesized when an electrical potential is applied to electrodes comprising an anode and a cathode, wherein the electrical potential is 1-5 volts, or is 2 volts or more, e.g., 3 volts or more, e.g., 4 volts or more.
1.11. Any foregoing product wherein persulfate is synthesized when an electrical potential is applied to electrodes comprising an anode and a cathode, wherein the electrical potential is about 3 volts.
1.12. Any foregoing product wherein persulfate is synthesized when an electrical potential is applied to electrodes comprising an anode and a cathode, wherein the electrical potential is about 4 volts.
1.13. Any foregoing product wherein persulfate is synthesized when an electrical potential is applied to electrodes comprising an anode and a cathode, wherein the persulfate is synthesized at the anode.
1.14. Any foregoing product wherein persulfate is synthesized when an electrical potential is applied to electrodes comprising an anode and a cathode, wherein hydrogen is synthesized at the cathode.
1.15. Any foregoing product wherein the product comprises an electrolyte.
1.16. Any foregoing product wherein the product comprises an electrolyte, wherein the electrolyte comprises water.
1.17. Any foregoing product wherein the product comprises an electrolyte, wherein the electrolyte comprises an inorganic salt.
1.18. Any foregoing product wherein the product comprises an electrolyte, wherein the electrolyte comprises one or more of sodium, potassium, lithium, calcium, strontium, and magnesium.
1.19. Any foregoing product wherein the product comprises an electrolyte, wherein the electrolyte comprises one or more of fluoride, chloride, bromide, or iodide, e.g., fluoride or chloride, e.g., chloride.
1.20. Any foregoing product wherein the product comprises an electrolyte, wherein the electrolyte is one or more of sodium chloride, potassium chloride, lithium chloride, calcium chloride, strontium chloride, and magnesium chloride, e.g., sodium chloride.
1.21. Any foregoing product wherein the product comprises an electrolyte, wherein the electrolyte is a buffer.
1.22. Any foregoing product wherein the product comprises an electrolyte, wherein the electrolyte comprises one or more of phosphate, citrate, acetate, lactate, and borate, e.g., phosphate.
1.23. Any foregoing product wherein the product comprises an electrolyte, wherein the electrolyte comprises one or more phosphoric acid, citric acid, acetic acid, lactic acid, and boric acid, e.g., phosphoric acid.
1.24. Any foregoing product wherein the product comprises an electrolyte, wherein the electrolyte comprises an amino acid, e.g., glutamic acid.
1.25. Any foregoing product, wherein the product further comprises one or more additional whitening agents, e.g., oxygen, ozone, a peroxide, e.g., hydrogen peroxide, a peracid, e.g., peracetic acid, or a chlorite, e.g., sodium chlorite, a hypochlorite, e.g., sodium hypochlorite.
1.26. Any foregoing product wherein the electrically conductive medium has an acidic pH, e.g, pH 4-6.5, e.g. about pH 5-6, for example, about pH 5.
1.27. Any foregoing product comprising electrodes comprising an anode and a cathode electrically connected to a voltage source, wherein the voltage source comprises a battery, e.g., a rechargeable battery, a capacitor, or an insulated wire comprising a plug.
1.28. Any foregoing product wherein the product comprises a tray, strip, or toothbrush, said tray, strip, or toothbrush comprising electrodes comprising an anode and a cathode electrically connectable to a voltage source, e.g., via a switch, e.g., such that persulfate can be generated in the mouth when the switch is turned on and an electrical potential is applied to the bisulfate in the buffered, electrically conductive, aqueous medium.
1.29. Any foregoing product comprising a container, wherein the container contains a sulfate (which in solution may form sulfate ion, bisulfate ion or an equilibrium between the two) in a buffered, electrically conductive, aqueous medium, and wherein the container comprises electrodes comprising an anode and a cathode electrically connectable to a voltage source, e.g., via a switch, e.g., such that persulfate can be generated in the container when the switch is turned on and an electrical potential is applied to the sulfate in the buffered, electrically conductive, aqueous medium, prior to application of the contents of the container to the teeth.
1.30. Product 1.29 wherein the container comprises a reservoir chamber with an aperture, which reservoir chamber contains the medium prior to use, and a cap covering the aperture, the cap comprising a measuring chamber with the electrodes being situated on the inner surface of the measuring chamber, such that the medium can be dispensed from the reservoir chamber into the measuring chamber in contact with the electrodes for activation prior to use.
1.31. Product 1.30 wherein the measuring chamber is connected to the reservoir chamber by a tube extending to the lower portion of the reservoir chamber.
1.32. Product 1.30 wherein the measuring chamber is connected to the reservoir chamber by a one-way valve, e.g. a ball valve, allowing flow into the measuring chamber when the product is inverted, such that when the product is no longer inverted, the cap is removed and inverted, then the valve is removed to permit the mouthwash to be administered to the user.
1.33. Product 1.31 wherein the tube is operably connected to a pump which can pump the medium from the reservoir chamber into the measuring chamber.
1.34. Product 1.31 wherein the walls of the reservoir chamber are deformable, such that when the walls are squeezed, the medium flows through the tube into the measuring chamber.
1.35. Any foregoing product wherein the buffered, electrically conductive medium further comprises an orally acceptable dye, e.g., lissamine green or a food dye, e.g., FD&C Blue #1, which provides a visual indication of the presence of persulfate, for example a water soluble dye which changes or loses color when oxidized, e.g., so that the medium correspondingly changes or loses color when the persulfate is generated.
1.36. Any foregoing product, wherein the product further comprises one or more flavoring agents (e.g., sucralose, sodium saccharin).
1.37. Any foregoing product, wherein the product further comprises one or more antimicrobial agents (e.g., benzyl alcohol, thymol).
1.38. Any foregoing product wherein the product is for whitening a tooth surface, or for oxidizing volatile sulfur compound that give rise to bad breath, or for killing bacteria on the teeth.
1.39. Any foregoing product, wherein one or both electrodes are nickel electrodes, optionally wherein both electrodes are nickel electrodes.
1.40. Product 1.39, wherein the nickel electrodes are cleaned, either before or after incorporation into the product, using a blasting process with a slurry of silicon carbide or aluminum oxide powder in water.
1.41. Any foregoing product, wherein the voltage is about 4V with a current maintained between 20 and 30 mA.
1.42. Any foregoing product, wherein the electrically conductive medium is a gel (e.g., an oral gel, e.g., an orally acceptable gel).
1.43. Any foregoing product, wherein the electrically conductive medium comprises a humectant, e.g., glycerin, sorbitol or propylene glycol, or a combination thereof; optionally where the humectant is present in an amount from 1-50 wt% of the medium, e.g., 5-40%, or 15-25%.
1.44. Any foregoing product, wherein the electrically conductive medium comprises an abrasive, e.g., a silica abrasive.
1.45. Any foregoing product, wherein the electrically conductive medium comprises the following ingredients:

| **Ingredient** | **Weight** % |
|---|---|
| GLYCERIN (e.g., 99.0% - 101.0%) | 15-25% |
| WATER | Q.S. |
| Sulfate salt (e.g., bisulfate salt, e.g., sodium bisulfate) | 1-10% |
| Electrolyte (e.g., citric acid) | 1-10%, e.g. 2-4% |
| Base (e.g. sodium hydroxide, e.g., 50 wt% Aq NaOH) | 1-10%, e.g. 2-4% |
| Polyacrylic acid polymer (e.g., Carbopol) | 0-5%, e.g. 0.5-2% |
| Silica (e.g., fumed silica) | 0-5%, e.g. 0.5-2% |
| Polysaccharide (e.g., xanthan gum, gellan gum, CMC) | 0-5%, e.g. 0.5-2% |

Further provided is a method (Method A) of providing an oral care composition comprising persulfate (S₂O₈²⁻) comprising applying an electrical potential to an oral care composition comprising a soluble and orally acceptable sulfate in a buffered, electrically conductive medium, e.g.,
A. 1. Method A wherein the method comprises oxidation of bisulfate (HSO₄⁻) or sulfate (SO₄²⁻).
A.2. Method A or A.1 wherein the electrical potential is applied using electrodes wherein the electrodes comprise a cathode and an anode.
A.3. Method A.2 wherein the medium comprises water.
A.4. Method A.2 or A.3 wherein the medium comprises an inorganic salt.
A.5. Any of Method A.2-A.4 wherein the medium comprises one or more of sodium, potassium, lithium, calcium, strontium, and magnesium.
A.6. Any of Method A.2-A.5 wherein the medium comprises one or more of fluoride, chloride, bromide, or iodide, e.g., fluoride or chloride, e.g., chloride.
A.7. Any of Method A.2-A.6 wherein the medium comprises an electrolyte selected from one or more of sodium chloride, potassium chloride, lithium chloride, calcium chloride, strontium chloride, and magnesium chloride, e.g., sodium chloride.
A.8. Any of Method A.2-A.7 wherein the buffer for the medium comprises one or more of phosphate, citrate, acetate, lactate, and borate, e.g., phosphate.
A.9. Any of Method A.2-A.9 wherein the medium comprises one or more phosphoric acid, citric acid, acetic acid, lactic acid, and boric acid, e.g., phosphoric acid.
A.10. Any of Method A.2-A. 10 wherein the medium comprises an amino acid, e.g., glutamic acid.
A.11. Any of Method A.2-A.11 wherein the electrodes comprise one or more conductive materials, e.g., a metal or polymer, e.g., wherein the pairs of electrodes comprise one or more of a conductive element, e.g., selected from carbon, platinum, iron, zinc, copper, aluminum, silver, nickel, and gold, e.g., one or more of platinum, carbon, and iron, e.g., platinum, e.g., carbon, e.g., iron, or wherein the one or more pairs of electrodes comprise a semiconductor material, e.g., titanium dioxide or boron doped diamond.
A.12. Any of Method A.2-A. 12 wherein the electrical potential is 1-5 volts, or 2 volts or more, e.g., 3 volts or more, e.g., 4 volts or more.
A.13. Any of Method A.2-A. 13 wherein the electrical potential is 3 volts.
A.14. Any of Method A.2-A. 13 wherein the electrical potential is 4 volts.
A.15. Any of Method A.2-A.15 wherein the current is 10-200 milliamps, e.g., 10-160 mA, or 10-120 mA, or 20-100 mA, or 20-80 mA, or 30-60 mA.
A.16. Any of Method A.2-A.16 wherein the persulfate is synthesized at the anode.
A.17. Any of Method A.2-A.17 wherein hydrogen is synthesized at the cathode.
A.18. Any of Method A.2-A.17 wherein the polarity of the electrodes alternates during use, e.g., to reduce build-up of charged particles or ions at one or the other electrode.
A. 19. Any of Method A.2-A.18, wherein one or both electrodes are nickel electrodes, optionally wherein both electrodes are nickel electrodes.
A.20. Method A. 19, wherein the nickel electrodes have been cleaned, either before or after incorporation into the product, using a blasting process with a slurry of silicon carbide or aluminum oxide powder in water.
A.21. Any of Method A.2-A.20, wherein the voltage is about 4V with a current maintained between 20 and 30 mA.
A.22. Any foregoing Method, wherein the method comprises applying an electrical potential to an oral care product of any of Product 1, *et seq.*

Further provided is a method (Method B) for whitening a tooth surface, or for oxidizing volatile sulfur compounds that give rise to halitosis, or for killing bacteria on a tooth surface, comprising
(a) applying an electrical potential to an oral care composition comprising a soluble orally acceptable sulfate in a buffered, electrically conductive medium (for example an oral care product which is any of Product 1, *et seq.)* to obtain an oral care composition comprising persulfate (S₂O₈²⁻), (e.g. in accordance with any of Method A, *et seq.*) and applying to the tooth surface an effective amount of the oral care composition comprising persulfate thus obtained, e.g., by brushing or rinsing, or
(b) applying to the teeth an oral care composition comprising a soluble orally acceptable sulfate in a buffered, electrically conductive medium (for example an oral care product which is any of Product 1, *et seq.),* and applying an electrical potential to said oral care composition to produce persulfate (S₂O₈²⁻) *in situ,* (e.g. in accordance with any of Method A, *et seq.*).
   e.g.
   B.1. Method B wherein the persulfate is synthesized by oxidation of sulfate (SO₄²⁻) or bisulfate (HSO₄⁻) ions.
   B.2. Method B or B.1 wherein the persulfate is synthesized by contacting sulfate with an electrolyte and applying an electrical potential to at least two electrodes comprising a cathode and an anode.
   B.3. Method B.2 wherein the electrolyte comprises water.
   B.4. Method B.2 or B.3 wherein the electrolyte comprises an inorganic salt.
   B.5. Any of Method B.2-B.4 wherein the electrolyte comprises one or more of sodium, potassium, lithium, calcium, strontium, and magnesium.
   B.6. Any of Method B.2-B.5 wherein the electrolyte comprises one or more of fluoride, chloride, bromide, or iodide, e.g., fluoride or chloride, e.g., chloride.
   B.7. Any of Method B.2-B.6 wherein the electrolyte is one or more of sodium chloride, potassium chloride, lithium chloride, calcium chloride, strontium chloride, and magnesium chloride, e.g., sodium chloride.
   B.8. Any of Method B.2-B.7 wherein the electrolyte is a buffer.
   B.9. Any of Method B.2-B.8 wherein the electrolyte comprises one or more of phosphate, citrate, acetate, lactate, and borate, e.g., phosphate.
   B.10. Any of Method B.2-B.9 wherein the electrolyte comprises one or more phosphoric acid, citric acid, acetic acid, lactic acid, and boric acid, e.g., phosphoric acid.
   B.11. Any of Method B.2-B. 10 wherein the electrolyte comprises an amino acid, e.g., glutamic acid.
   B.12. Any of Method B.2-B. 11 wherein the pair of electrodes comprises one or more conductive materials, e.g., a metal or polymer, e.g., wherein the pairs of electrodes comprise one or more of a conductive element, e.g., selected from carbon, platinum, iron, zinc, copper, aluminum, silver, nickel, and gold, e.g., one or more of platinum, carbon, and iron, e.g., platinum, e.g., carbon, e.g., iron, or wherein the one or more pairs of electrodes comprise a semiconductor material, e.g., titanium dioxide or boron doped diamond.
   B.13. Any of Method B.2-B.12 wherein the electrical potential is 1-5 volts, or 2 volts or more, e.g., 3 volts or more, e.g., 4 volts or more.
   B.14. Any of Method B.2-B.13 wherein the electrical potential is about 3 volts.
   B.15. Any of Method B.2-B.13 wherein the electrical potential is about 4 volts.
   B.16. Any of Method B.2-B.15 wherein the current applied is 10-200 milliamps, e.g., 10-160 mA, or 10-120 mA, or 20-100 mA, or 20-80 mA, or 30-60 mA.
   B. 17. Any of Method B.2-B. 16 wherein the persulfate is synthesized at the anode.
   B.18. Any of Method B.2-B.17 wherein hydrogen is synthesized at the cathode.
   B.19. Any of Method B.2-B.18 wherein the polarity of the electrodes alternates during use, e.g., to reduce build up of charged particles or ions at one or the other electrode.
   B.20. Any of Method B.2-B. 19, wherein one or both electrodes are nickel electrodes, optionally wherein both electrodes are nickel electrodes.
   B.21. Method B.20, wherein the nickel electrodes have been cleaned, either before or after incorporation into the product, using a blasting process with a slurry of silicon carbide or aluminum oxide powder in water.
   B.22. Any of Method B.2-B.21, wherein the voltage is about 4V with a current maintained between 20 and 30 mA.

It is surprising that stable non-bleaching salts like sodium bisulfate can be made useful in methods of bleaching teeth, oxidizing volatile compounds and killing bacteria. Further provided therefore, is the use (Use A) of sulfate salt, e.g., selected from sodium bisulfate, potassium bisulfate, ammonium bisulfate, magnesium bisulfate, sodium sulfate, potassium sulfate, ammonium sulfate, magnesium sulfate, and mixtures thereof (for example in a formulation according to any of Product 1, *et seq.*) for whitening a tooth surface, or for oxidizing volatile sulfur compounds that give rise to halitosis, or for killing bacteria on a tooth surface, e.g., in accordance with any of Methods B, *et seq.*

### EXAMPLES

### Example 1- Electrochemical synthesis of persulfates

Scheme 1 illustrates the electrochemical synthesis of potassium persulfate form potassium bisulfate.

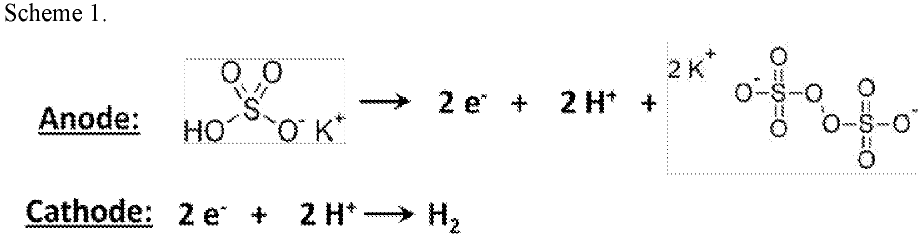

Potassium bisulfate (HKSO₄) at concentrations of 1-10%, by weight is dissolved in a phosphate buffer and the pH is adjusted as necessary for Lissamine Green bleaching experiments. An anode and cathode electrode is submersed in the reaction vessel and the generation of the potassium persulfate salt as a function of potassium bisulfate concentration, pH, voltage / current, and electrode material is monitored using Lissamine Green absorbance changes. The decrease of the absorbance (610 nm) of Lissamine Green over time is indicative of its bleaching by the persulfate produced. Alternatively, platinum electrodes are used and potassium bisulfate is added at 10% to distilled water, the reaction vessel is cooled to 4°C, and potassium persulfate powder is collected and confirmed with IR and XPS.

While the potassium persulfate has bleaching activity over a range of pH, the activity is greater at acidic pH.

| **Table 1**. pH dependence on in situ bleaching of lissamine green by potassium persulfate produced electrochemically from potassium bisulfate solution using platinum electrodes. | | | |
|---|---|---|---|
| Time (min) | Absorbance at pH 6 | Absorbance at pH 7 | Absorbance at pH 8 |
| 0 | 1 | 1 | 1 |
| 2 | 0.637 | 0.885 | 0.843 |
| 4 | 0.508 | 0.770 | 0.758 |
| 6 | 0.390 | 0.686 | 0.68 |
| 8 | 0.253 | 0.599 | 0.58 |
| 10 | 0.203 | 0.520 | 0.55 |
| 12 | 0.148 | 0.412 | 0.5 |
| 14 | 0.126 | 0.388 | 0.483 |
| 16 | 0.103 | 0.315 | 0.409 |
| 18 | 0.091 | 0.295 | 0.376 |
| 20 | 0.081 | 0.296 | 0.358 |

Different electrodes, including carbon, platinum, iron, zinc, copper, and aluminum, are tested:

| **Table 2**. 3 Volts applied bias at 5% HKSO₄, using multiple electrode materials | | | | | |
|---|---|---|---|---|---|
| Time (min) | Platinum | Carbon | Aluminum | Iron | Copper |
| 0 | 1 | 1 | 1 | 1 | 1 |
| 2 | 0.964 | 1 | 1.003 | 0.95 | 0.990 |
| 4 | 0.884 | 0.957 | 0.975 | 0.89 | 0.955 |
| 6 | 0.72 | 0.944 | 0.987 | 0.86 | 0.951 |
| 8 | 0.636 | 0.871 | 0.971 | 0.774 | 0.924 |
| 10 | 0.60 | 0.791 | 0.999 | 0.77 | 0.93 |
| 12 | 0.580 | 0.753 | 0.966 | 0.724 | 0.93 |
| 14 | 0.510 | 0.700 | 0.929 | 0.68 | 0.935 |
| 16 | 0.397 | 0.635 | 0.974 | 0.65 | 0.933 |
| 18 | 0.384 | 0.671 | 0.922 | 0.62 | 0.898 |
| 20 | 0.374 | 0.582 | 0.922 | 0.57 | 0.875 |

Although they all show the capability of producing the active, platinum, carbon, and iron are preferred. Zinc is also tested, but performs poorly. Two more electrodes-nickel and Boron Doped Diamond Electrodes-are tested using gels and perform very well.

The effect of voltage using different electrodes is assessed:

| **Table 3.** Comparison of bleaching between platinum, carbon, and iron electrodes at 3 Volts versus 4 Volts applied bias at 5% HKSO₄ | | | | | | |
|---|---|---|---|---|---|---|
| Time (min) | Platinum (3V) | Carbon (3V) | Iron (3V) | Platinum (4V) | Carbon (4V) | Iron (4V) |
| 0 | 1 | 1 | 1 | 1 | 1 | 1 |
| 2 | 0.964 | 1 | 0.952 | 0.89 | 0.979 | 0.951 |
| 4 | 0.884 | 0.957 | 0.897 | 0.810 | 0.922 | 0.85 |
| 6 | 0.720 | 0.9445 | 0.865 | 0.671 | 0.806 | 0.76 |
| 8 | 0.6360 | 0.871 | 0.774 | 0.617 | 0.67 | 0.69 |
| 10 | 0.600 | 0.7919 | 0.776 | 0.53 | 0.63 | 0.64 |
| 12 | 0.5809 | 0.753 | 0.724 | 0.491 | 0.638 | 0.59 |
| 14 | 0.510 | 0.700 | 0.6837 | 0.434 | 0.581 | 0.55 |
| 16 | 0.397 | 0.635 | 0.654 | 0.397 | 0.532 | 0.493 |
| 18 | 0.384 | 0.671 | 0.621 | 0.341 | 0.509 | 0.467 |
| 20 | 0.374 | 0.582 | 0.57 | 0.283 | 0.493 | 0.428 |

Effect at varying concentrations is also assessed:

| **Table 4.** Bleaching as a function of % HKSO4 | | | | |
|---|---|---|---|---|
| Time (min) | 1% HKSO4, pH 4 | 3% HKSO4, pH 4 | 7% HKSO4, pH 4 | 10% HKSO4, pH 4 |
| 0 | 1.000 | 1.000 | 1.000 | 1.000 |
| 2 | 0.985 | 0.968 | 0.864 | 0.851 |
| 4 | 0.914 | 0.854 | 0.779 | 0.746 |
| 6 | 0.830 | 0.754 | 0.673 | 0.646 |
| 8 | 0.740 | 0.679 | 0.579 | 0.535 |
| 10 | 0.635 | 0.582 | 0.496 | 0.470 |
| 12 | 0.559 | 0.512 | 0.427 | 0.407 |
| 14 | 0.502 | 0.456 | 0.370 | 0.355 |
| 16 | 0.432 | 0.412 | 0.322 | 0.304 |
| 18 | 0.425 | 0.360 | 0.270 | 0.256 |
| 20 | 0.396 | 0.331 | 0.238 | 0.225 |

### Example 2: Efficacy on stained teeth

Whitening efficacy on bovine teeth is assessed. Bovine teeth are brushed to achieve similar initial lightness values. The bovine teeth are placed in artificial saliva (composed up of 7.5 mL of Mucin solution and 7.5 mL of Saliva Buffer) for 10 minutes. Each sample is treated using electrochemistry treatment for 15-20 minutes in a phosphate-based buffer solution. The platinum electrodes are placed 2 mm from the bovine tooth surface. The electrode area is 1.2 cm x 1.2 cm and the bovine teeth are roughly 0.5 cm x 0.5 cm, and the applied bias is 4 volts. After treatment, the bovine teeth are rinsed with deionized water and the color (L*a*b* values) is measured with a spectrophotometer.

The spectrophotometer used is Spectroshade from MHT. The measurement scale is the CIE L*a*b* (CIELAB) scale developed by the International Commission on Illumination (CIE). CIELAB is an opponent color system based on the fact that retinal color stimuli are translated into distinctions between light and dark, red and green, and blue and yellow. CIELAB indicates these values with three axes: L*, a*, and b*. The L value indicates the lightness of a color, where L=0 is black and L=100 is white. ΔL = L_{treated} - Lᵢₙᵢₜᵢₐₗ. Thus, a larger positive ΔL value = whiter teeth. The a value ranges between +a = magenta and -a = green. The b value ranges between +b = yellow and -b = blue. The W value incorporates the L, a and b values to describe how close the measured color is to true white, where W* = (a2 + b2 + (L*-100)2)1/2. A larger negative ΔW value corresponds to greater whitening.

A comparison among 10% HKSO₄ with no power applied (control); 10% HKSO₄, activated with platinum electrodes at 4 volts (current ∼100 mA); and 4.5% H₂O₂ gel, in this assay is as follows (measurements for each treatment taken at approx. 20 minute intervals):

| **Table 5.** | | | |
|---|---|---|---|
| Treatment | ΔW | ΔW | ΔW |
| | HKSO₄, no power | HKSO₄ + 4 Volts | 4.5% H₂O₂ |
| 0 | 0 | 0 | 0 |
| 1 | -1.663 | -5.11 | -1.947 |
| 2 | -1.947 | -7.451 | -3.616 |
| 3 | -1.927 | -9.498 | -4.162 |
| 4 | -2.06 | -10.194 | -5.007 |
| 5 | -2.294 | -10.86 | -5.973 |

The generated persulfate is thus able to travel through the solution and bleach a stained bovine tooth significantly more quickly and completely than 4.5% H₂O₂ gel.

The bleaching of the stained bovine teeth using different electrodes is assessed.

| **Table 6.** 4 Volts applied bias at 10% HKSO₄, looking at different electrode materials | | | | |
|---|---|---|---|---|
| Treatment | ΔW | ΔW | ΔW | ΔW |
| | Steel Anode | Nickel Anode | Pt Electrode | BDD Electrode |
| 1 | -0.749 | -3.828 | -5.11 | -1.718 |
| 2 | -0.717 | -5.438 | -7.45 | -2.216 |
| 3 | -0.723 | -6.086 | -9.498 | -3.199 |
| 4 | -0.6947 | -6.44 | -10.19 | -3.78 |
| 5 | -0.7418 | -6.927 | -10.87 | -4.02 |

The efficacy of sodium bisulfate solution vs. potassium bisulfate solution for whitening bovine teeth is about the same:

| **Table 7.** Comparing persulfate whitening efficacy as a function of counter-ion: Sodium (Na⁺) versus Potassium (K⁺) with 4 Volts applied bias, 10% solution | | |
|---|---|---|
| Treatment | ΔW | ΔW |
| | HNaSO4 | HKSO4 |
| 0 | 0 | 0 |
| 1 | -3.126 | -5.110 |
| 2 | -6.915 | -7.451 |
| 3 | -9.875 | -9.498 |

### Example 3: Bisulfate gel formulations

Sodium bisulfate is much more soluble than potassium bisulfate, and so is preferred for use in gel formulations. Different gel formulations are prepared and tested, and a citrate buffer was used instead. Gels are in the pH 5-6 range. Generally, gels have a composition such as:

| **Formulation A** | |
|---|---|
| **Ingredient** | **Weight %** |
| 99.0% - 101.0% GLYCERIN | 16-24%, e.g., 18-19% |
| DEMINERALIZED WATER | Q.S. |
| Sodium bisulfate | 5-15%, e.g. 10% |
| Citric Acid | 1-3%, e.g., 1.9% |
| SODIUM HYDROXIDE (50%) | 6-8%, e.g., 7.0% |
| Carbopol (e.g., Carbomer 974P) | 0-2%, e.g., 0.3-0.8%, e.g., 0.6% |
| Linear-PVP (e.g., Plasdone K90) | 0-5%, e.g., 1-2% |
| Xanthan Gum | 0.1-5%, e.g, 0.5-2%, e.g., 0.5-1% |
| Silica (e.g., fumed silica) | 0-5%, e.g., 0.5-2%, e.g., 0.5-1% |
| Flavoring (e.g., sweeteners) | 0-1% |
| Antimicrobial agent or preservative (e.g., benzyl alcohol) | 0-2%, e.g., 0.5-1% |

Three particular candidate formulations are evaluated:

| **Formulation 1** | |
|---|---|
| **Ingredient** | **Weight %** |
| 99.0% - 101.0% GLYCERIN - USP, EP VEG | 18.00% |
| DEMINERALIZED WATER | 62.18% |
| Sodium bisulfate | 10.00% |
| Citric Acid | 1.92% |
| SODIUM HYDROXIDE -50% (REAGENT GRADE) | 7.00% |
| Carbopol | 0.50% |
| Xanthan Gum | 0.40% |

| **Formulation 2** | |
|---|---|
| **Ingredient** | **Weight %** |
| 99.0% - 101.0% GLYCERIN - USP, EP VEG | 17.25% |
| DEMINERALIZED WATER | 62.18% |
| Sodium bisulfate | 10.00% |
| Citric Acid | 1.92% |
| SODIUM HYDROXIDE -50% (REAGENT GRADE) | 7.00% |
| Carbopol | 0.50% |
| Fumed Silica | 0.75% |
| Xanthan Gum | 0.40% |

| **Formulation 3** | |
|---|---|
| **Ingredient** | **Weight %** |
| 99.0% - 101.0% GLYCERIN - USP, EP VEG | 17.00% |
| DEMINERALIZED WATER | 62.18% |
| Sodium bisulfate | 10.00% |
| Citric Acid | 1.92% |
| SODIUM HYDROXIDE -50% (REAGENT GRADE) | 7.00% |
| Carbopol | 0.50% |
| L-PVP (Plasdone K90) | 1.00% |
| Xanthan Gum | 0.40% |

These gels are tested in the bovine staining assay:

| **Table 8.** Three sample gels prepared and their bleaching efficacy at 4V using Pt electrodes | | | |
|---|---|---|---|
| Treatment | ΔW | ΔW | ΔW |
| | Gel Formulation 1 | Gel Formulation 2 | Gel Formulation 3 |
| 0 | 0 | 0 | 0 |
| 1 | -0.498692738 | -0.6427121 | -0.39935 |
| 2 | -1.196476062 | -1.6506523 | -0.7937 |
| 3 | -1.497749457 | -1.9713629 | -1.08873 |
| 4 | -1.786314714 | -2.6691878 | -1.34656 |
| 5 | -2.110957569 | -2.873121 | -1.57661 |

Gels containing cross-linked PVP, carboxymethyl cellulose and gellan gum are also tested with varying degrees of success. The gels above contain persulfate with the sodium cation, but the potassium is also tested, with successful bleaching results.

### Example 4: Aluminum Oxide Blast-Cleaned Nickel Electrodes

In order to evaluate the effect of different electrode cleaning methods on the effectiveness of nickel electrodes, an experiment is conducted using cleaned nickel electrodes. One set of electrodes are cleaned by blasting with distilled water. The other set of electrodes are cleaned by blasting with a slurry of aluminum oxide powder in distilled water. Formulation 4 was used as the gel, and each treatment was conducted for about 20 minutes with a voltage of 4V and a current fluctuating between 20 and 30 mA. The pH of the formulation was 5. As shown in the table below, it is found that the alumina-cleaned electrodes perform much better than water-cleaned electrodes. It is believed this is due to the stripping of nickel oxide and other impurities from the surface of the nickel electrode, as well due to the creation of a microscopically rough surface which provides better electrical contact to the gel.

| Treatment | ΔW, Water Cleaned | ΔW, Alumina Cleaned |
|---|---|---|
| 0 | 0 | 0 |
| 1 | -5.096 | -1.688 |
| 2 | -9.855 | -4.823 |
| 3 | -10.754 | -7.991 |
| 4 | -11.789 | -10.485 |
| 5 | -12.769 | -11.587 |
| 6 | -14.223 | -12.382 |

| **Formulation 4** | |
|---|---|
| **Ingredient** | **Weight %** |
| 99.0% - 101.0% GLYCERIN - USP, EP VEG | 19.08% |
| DEMINERALIZED WATER | 60.00%. |
| Sodium bisulfate | 10.00% |
| Citric Acid | 1.92% |
| SODIUM HYDROXIDE -50% (REAGENT GRADE) | 7.00% |
| Carbopol | 0.60% |
| Fumed Silica | 0.80% |
| Xanthan Gum | 0.60% |

## Claims

1. An oral care product comprising an effective amount of a soluble orally acceptable sulfate in a buffered, electrically conductive medium, wherein the product comprises at least two electrodes electrically connectable to a voltage source, wherein the electrodes comprise a cathode and an anode.

2. The oral care product of claim 1, wherein the sulfate is selected from sodium bisulfate, potassium bisulfate, ammonium bisulfate, magnesium bisulfate, sodium sulfate, potassium sulfate, ammonium sulfate, magnesium sulfate, and mixtures thereof.

3. The oral care product of claim 2 wherein the sulfate is sodium bisulfate.

4. The oral care product of any foregoing claim wherein the buffered, electrically conductive medium is an orally acceptable aqueous gel or an orally acceptable aqueous salt solution.

5. The oral care product of claim 1, wherein the electrodes comprise one or more of platinum, carbon, iron, nickel, or a semiconductor material.

6. The oral care product of claim 1 or 5, wherein the polarity of the electrodes alternates during use.

7. The oral care product of claim 1, 5, or 6 wherein the electrodes are provided in a container cap, or a tray, or a toothbrush.

8. The oral care product of any foregoing claim, wherein persulfate is synthesized when an electrical potential is applied to the sulfate in a buffered, electrically conductive medium.

9. The oral care product of any foregoing claim wherein the buffered, electrically conductive medium comprises an electrolyte.

10. The oral care product of any foregoing claim further comprising an orally acceptable dye which provides a visual signal when persulfate is present.

11. The oral care product of any foregoing claim, wherein the product comprises one or more additional whitening agents.

12. The oral care product of any foregoing claim comprising a voltage source comprising a battery, a capacitor, or an insulated wire comprising a plug.

13. A method of providing an oral care composition comprising persulfate (S₂O₈²⁻) , comprising applying an electrical potential to an oral care composition comprising a soluble orally acceptable sulfate in a buffered, electrically conductive medium.

14. An oral care product for use in a method for whitening a tooth surface, or for oxidizing volatile sulfur compounds that give rise to halitosis, or for killing bacteria on a tooth surface, comprising applying an electrical potential to an oral care composition comprising a soluble orally acceptable sulfate in a buffered, electrically conductive medium to obtain an oral care composition comprising persulfate (S₂O₈²⁻ ), and applying to the tooth surface an effective amount of the oral care composition comprising persulfate thus obtained.

## Patentansprüche

1. Mundpflegeprodukt, umfassend eine wirksame Menge eines löslichen, oral annehmbaren Sulfats in einem gepufferten, elektrisch leitfähigen Medium, wobei das Produkt mindestens zwei Elektroden umfasst, die elektrisch mit einer Spannungsquelle verbunden werden können, wobei die Elektroden eine Kathode und eine Anode umfassen.

2. Mundpflegeprodukt nach Anspruch 1, worin das Sulfat ausgewählt ist aus Natriumbisulfat, Kaliumbisulfat, Ammoniumbisulfat, Magnesiumbisulfat, Natriumsulfat, Kaliumsulfat, Ammoniumsulfat, Magnesiumsulfat und Mischungen davon.

3. Mundpflegeprodukt nach Anspruch 2, wobei das Sulfat Natriumbisulfat ist.

4. Mundpflegeprodukt nach irgendeinem vorhergehenden Anspruch, worin das gepufferte, elektrisch leitfähige Medium ein oral annehmbares, wässriges Gel oder eine oral annehmbare, wässrige Salzlösung ist.

5. Mundpflegeprodukt nach Anspruch 1, wobei die Elektroden eines oder mehrere von Platin, Kohlenstoff, Eisen, Nickel oder ein Halbleitermaterial umfassen.

6. Mundpflegeprodukt nach Anspruch 1 oder 5, wobei die Polarität der Elektroden während der Verwendung abwechselt.

7. Mundpflegeprodukt nach Anspruch 1, 5 oder 6, wobei die Elektroden in einem Behälterverschluss, einer Schale oder einer Zahnbürste bereitgestellt werden.

8. Mundpflegeprodukt nach irgendeinem vorhergehenden Anspruch, worin Persulfat synthetisiert wird, wenn ein elektrisches Potential auf das Sulfat in einem gepufferten, elektrisch leitfähigen Medium aufgebracht wird.

9. Mundpflegeprodukt nach irgendeinem vorhergehenden Anspruch, worin das gepufferte, elektrisch leitfähige Medium einen Elektrolyten umfasst.

10. Mundpflegeprodukt nach irgendeinem vorhergehenden Anspruch, umfassend einen oral annehmbaren Farbstoff, der ein visuelles Signal bereitstellt, wenn Persulfat vorliegt.

11. Mundpflegeprodukt nach irgendeinem vorhergehenden Anspruch, worin das Produkt ein oder mehrere zusätzliche Aufhellungsmittel umfasst.

12. Mundpflegeprodukt nach irgendeinem vorhergehenden Anspruch, umfassend eine Spannungsquelle, umfassend eine Batterie, einen Kondensator oder einen isolierten Draht, der einen Stecker umfasst.

13. Verfahren zur Bereitstellung einer Mundpflegezusammensetzung, umfassend Persulfat (S₂O₈²⁻), umfassend das Aufbringen eines elektrischen Potentials auf eine Mundpflegezusammensetzung, umfassend ein lösliches, oral annehmbares Sulfat in einem gepufferten, elektrisch leitfähigen Medium.

14. Mundpflegeprodukt zur Verwendung in einem Verfahren zur Aufhellung einer Zahnoberfläche, oder zum Oxidieren von flüchtigen Schwefelverbindungen, die Halitosis auslösen können, oder zum Abtöten von Bakterien auf einer Zahnoberfläche, umfassend das Aufbringen eines elektrischen Potentials auf eine Mundpflegezusammensetzung, umfassend ein lösliches, oral annehmbares Sulfat in einem gepufferten, elektrisch leitfähigen Medium, um eine Mundpflegezusammensetzung zu erhalten, die Persulfat (S₂O₈²⁻) umfasst, und Aufbringen einer wirksamen Menge der so erhaltenen Mundpflegezusammensetzung, umfassend Persulfat, auf die Zahnoberfläche.

## Revendications

1. Produit de soin buccal comprenant une quantité efficace d'un sulfate acceptable par voie orale, soluble, dans un milieu conducteur de l'électricité, tamponné, dans lequel le produit comprend au moins deux électrodes aptes à être connectées électriquement à une source de tension, les électrodes comprenant une cathode et une anode.

2. Produit de soin buccal selon la revendication 1, dans lequel le sulfate est choisi parmi le bisulfate de sodium, le bisulfate de potassium, le bisulfate d'ammonium, le bisulfate de magnésium, le sulfate de sodium, le sulfate de potassium, le sulfate d'ammonium, le sulfate de magnésium et leurs mélanges.

3. Produit de soin buccal selon la revendication 2, dans lequel le sulfate est le bisulfate de sodium.

4. Produit de soin buccal selon l'une quelconque des revendications précédentes, dans lequel le milieu conducteur de l'électricité, tamponné, est un gel aqueux acceptable par voie orale ou une solution aqueuse de sel acceptable par voie orale.

5. Produit de soin buccal selon la revendication 1, dans lequel les électrodes comprennent au moins l'un parmi le platine, le carbone, le fer, le nickel ou un matériau semi-conducteur.

6. Produit de soin buccal selon l'une des revendications 1 ou 5, dans lequel la polarité des électrodes alterne pendant l'utilisation.

7. Produit de soin buccal selon l'une des revendications 1, 5 ou 6, dans lequel les électrodes sont disposées dans un bouchon de récipient, ou un plateau, ou une brosse à dents.

8. Produit de soin buccal selon l'une quelconque des revendications précédentes, dans lequel un persulfate est synthétisé lorsqu'un potentiel électrique est appliqué au sulfate dans un milieu conducteur de l'électricité, tamponné.

9. Produit de soin buccal selon l'une quelconque des revendications précédentes, dans lequel le milieu conducteur de l'électricité, tamponné, comprend un électrolyte.

10. Produit de soin buccal selon l'une quelconque des revendications précédentes, comprenant en outre un colorant acceptable par voie orale qui fournit un signal visuel lorsqu'un persulfate est présent.

11. Produit de soin buccal selon l'une quelconque des revendications précédentes, dans lequel le produit comprend un ou plusieurs agents de blanchiment supplémentaires.

12. Produit de soin buccal selon l'une quelconque des revendications précédentes, comprenant une source de tension comprenant une batterie, un condensateur ou un fil électrique isolé comprenant une prise.

13. Procédé pour fournir une composition de soin buccal comprenant du persulfate (S2O82-), comprenant l'application d'un potentiel électrique à une composition de soin buccal comprenant un sulfate acceptable par voie orale, soluble, dans un milieu conducteur de l'électricité, tamponné.

14. Produit de soin buccal pour une utilisation dans un procédé pour le blanchiment d'une surface dentaire, ou pour l'oxydation de composés volatils du soufre qui donnent naissance à une halitose, ou pour détruire des bactéries sur une surface dentaire, comprenant l'application d'un potentiel électrique à une composition de soin buccal comprenant un sulfate acceptable par voie orale, soluble, dans un milieu conducteur de l'électricité, tamponné, pour obtenir une composition de soin buccal comprenant du persulfate (S2O82-), et l'application à la surface dentaire d'une quantité efficace de la composition de soin buccal comprenant le persulfate ainsi obtenu.
